# EUROPEAN PATENT APPLICATION

(11) **EP 4 260 811 A1**
(43) Date of publication of application: **18.10.2023**
(21) Application number: 22176115.8
(22) Date of filing: 30.05.2022
(51) Int. Cl.: A61B 8/00, G16H 70/20

(54) **GRAPHICAL USER INTERFACE FOR PROVIDING ULTRASOUND IMAGING GUIDANCE**

(30) Priority: 12.04.2022 US 202263330012 P
(71) Applicant: Koninklijke Philips N.V., 5656 AG Eindhoven (NL)
(72) Inventor: HAVERSTOCK, Christopher, Edward, Eindhoven (NL); XUN, Mandy, Eindhoven (NL); FINCKE, Jonathan, 5656AG Eindhoven (NL); PATEL, Payaal, 5656AG Eindhoven (NL); WILSON, Martha, Gail, Grewe, Eindhoven (NL); CHEN, Alvin, Eindhoven (NL); PANSE, Ashish, Sattyavrat, Eindhoven (NL); LEE, Hyeon, Woo, Eindhove (NL)
(74) Representative: Philips Intellectual Property & Standards

(57) **Abstract**

A system (100) and method for providing guidance in an ultrasound imaging procedure involves presenting graphical user interface (131) elements overlaid onto an external image of the subject's body. The system includes at least one camera (137) for capturing the external image of the subject. The system may utilize trained artificial neural network(s) (150) to identify locations in the external image that correspond to scanning zones of a scanning protocol. A probe (112) placement marker is overlaid at each identified location to indicate the position of the probe's face onto the subject's body for acquiring a target view of the internal anatomy. Additional graphical elements may be presented via the guidance interface (133) and results findings may optionally be overlaid onto the external image of the subject at the completion of the scan. In embodiments herein, imaging guidance is provided solely via the external images of the subject without displaying the ultrasound image data.

## Description

### FIELD OF THE INVENTION

The present disclosure relates generally to ultrasound imaging and, in particular, to graphical user interfaces for guiding ultrasound imaging procedures based on obtained external images and optionally ultrasound image data of the patient.

### BACKGROUND OF THI INVENTION

Ultrasound imaging is one of a number of techniques for obtaining medical images of internal anatomical structures of a patient. Ultrasound imaging systems typically include an ultrasound transducer probe, which includes a transducer array supported by (e.g., enclosed in) a probe housing. The transducer array is activated to vibrate at ultrasonic frequencies to transmit ultrasonic energy into the patient's anatomy, and then receive ultrasonic echoes reflected or backscattered by the patient's anatomy to create an image. Such transducer arrays may include various layers, including some with piezoelectric materials, which vibrate in response to an applied voltage to produce the desired pressure waves. These transducers may be used to successively transmit and receive several ultrasonic pressure waves through the various tissues of the body. The various ultrasonic responses may be further processed by an ultrasonic imaging system to display the various structures and tissues of the body. The ultrasound imaging system may use a variety of imaging modes, such as B-mode and Doppler flow. For B-mode imaging, the ultrasound imaging system may create two-dimensional images of tissue in which the brightness of a pixel is based on the intensity of the reflected echo. For Doppler flow imaging, the ultrasound system may determine the movement of fluid (e.g., blood) or tissue based on a Doppler effect, where the reflected echoes are shifted in frequency with respect to the incident wave.

Recently, point-of-care ultrasound (POCUS) has become a standard tool used in an emergency department by the emergency physician(s), although the term POCUS is a broad term that includes many scenarios such as emergency room, ambulance, helicopter, etc., in which portable ultrasound can be used. There are a number of established protocols such as the Focused Assessment with Sonography in Trauma (FAST) for trauma, Bedside Lung Ultrasound in Emergency (BLUE) and Rapid Assessment of Dyspnea with Ultrasound (RADiUS) for dyspnea, Rapid Ultrasound in Shock (RUSH) for shock, and Focused Echocardiography in Emergency Life support (FEEL) for cardiac arrest. One difference between POCUS and consultative ultrasonography is that the doctor performs all acquisitions and may also interpret the images at the point of care, using the information immediately to address specific hypotheses and guide therapy in progress. For example, the FAST protocol is a rapid bedside ultrasound examination performed by surgeons, emergency physicians, and paramedics as a screening test for blood around the heart (pericardial effusion) or abdominal organs (hemoperitoneum) after trauma. In a FAST exam, the four areas that are typically examined for free fluid are the perihepatic space, the perisplenic space, the pericardium, and the pelvis to identify the presence of intraperitoneal or pericardial free fluid, which in the context of trauma will usually be due to bleeding. A variation of the FAST protocol is the extended focused assessment with sonography for trauma (eFAST), which allows the emergency physician or a surgeon the ability to determine whether a patient has pneumothorax, hemothorax, pleural effusion, mass/tumor, or a lodged foreign body.

Training physicians and maintaining their scanning skill in POCUS is an existing challenge to realizing the widespread use of ultrasound across the care continuum. In the context of emergency care, the FAST exam is one the exams known to suffer from the most sever fluctuations in accuracy (60% - 100%) depending on the medical professional executing the scan protocol. The accuracy of the exams depends on the number of exams the physician operator has done in the past and the time from their last exam. In other words, the FAST exam is a skill that requires practice to develop and the skill level decays with time if it is not practiced and new users of the exam are worse than experienced users. Moreover, when a patient arrives with a blunt trauma, the emergency room team and the trauma surgery team have minutes to diagnose if the patient has internal bleeding with the FAST exam and eventually CT scans. Diagnosis of internal bleeding will lead to emergent life-saving treatment, or careful observation. Currently, if a patient is stable the standard of care is to use CT for this diagnosis, but if the patient is unstable, the FAST exam is used to for diagnosis. The variability in skill of users is a major reason why FAST exams are not always effective, and a poor exam could delay life-saving treatment for the patient. Accordingly, new tools, such as ultrasound scan guidance, for improve the accuracy of POCUS may be desirable.

### SUMMARY OF THE INVENTION

While existing ultrasound imaging has proved useful for clinical examinations and diagnosis, there remains a need for improved guidance systems and techniques for example in the context of POCUS.

The invention is defined by the independent claims. The dependent claims define advantageous embodiments.

Aspects of the present disclosure provide guidance for ultrasound imaging systems, and associated devices and methods. Disclosed embodiments describe apparatuses, systems and methods for providing guidance in an ultrasound imaging procedure by overlaying and presenting graphical user interface (GUI) elements onto an external image of the patient's body. In embodiments herein, the user utilizes the external image of the patient's body as direct visual guidance rather than the ultrasound images produced by the imaging system. As such, the guidance system in effect provides an augmented reality interface in which the actual (captured) external image (e.g., video) of the patient is enhanced or augmented, in real time, to provide the guidance for assisting the user in performing the imaging procedure. The guidance system includes at least one camera for capturing the external image of the patient. The system may utilize trained artificial neural network(s) to identify locations in the external image that correspond to scanning zones associated with a given scanning protocol (e.g., FAST). A probe placement marker is overlaid at each identified location to indicate the position of the probe's face onto the patient's body for acquiring a target view of the internal anatomy. Additional graphical elements may be presented via the guidance interface, such as probe alignment graphics, a status/progress (or timer) graphic, a pressure graphic, etc. and, optionally, results findings graphics may be overlaid onto the external image of the patient at the completion of the scan. The results findings may provide a clear visual summary of the results of the scan and, optionally, enable the quick retrieval of the relevant underlying medical images based on which the findings were determined by the system.

In accordance with some embodiments, a method for providing guidance for an ultrasound imaging procedure involves acquiring an external image of a patient with a camera of a computing device. The computing device is communicatively coupled to an ultrasound imaging device including a probe. The method further involves identifying, in the external image, respective locations, each associated with one or more acoustic windows for an ultrasound imaging protocol. In some embodiments, the method further involves displaying, on a graphical user interface (GUI) of the computing device, the external image overlaid with a corresponding scanning zone label for each of the respective locations and for each of the respective locations. In some embodiments, upon selection of a given location, the method further involves sequentially displaying: (1) a probe placement graphic corresponding to a position of the probe's face onto the patient's body for acquiring a target view in the corresponding acoustic window, and (2) upon detecting acoustic coupling between the probe and the patient, at least one scanning guidance graphic which is updated in real-time based, in part, on live ultrasound image data acquired by the ultrasound imaging device.

In some embodiments, each of the scanning zone labels is a selectable graphical element of the graphical user interface configured that enables the user to select the corresponding location to initiate said sequential displaying. In some embodiments, the method further involves including animating the probe placement graphic to indicate at least one of: acoustic coupling between the probe and the patient, and alignment of the probe's field of view with the target view. In some embodiments, the animating of the probe placement graphic includes: (1) changing a color of the probe placement graphic upon detecting acoustic coupling between the probe and the patient or upon detecting alignment of the probe's field of view with the target view, (2) pulsating the probe placement graphic until alignment of the probe's field of view with the target view has been detected, or a combination of (1) and (2).

In some embodiments, the method further includes determining whether an expected volume of ultrasound image data from a current acoustic window has been recorded, and wherein the at least one scanning guidance graphic includes a progress indicator graphic animated to indicate a status of the recording of the expected volume. In some embodiments, the progress indicator graphic includes a circular progress bar encircling the probe placement marker in the external image. In some embodiments, the at least one scanning guidance graphic further includes a pressure indicator graphic displayed onto the external image adjacent to the progress indicator graphic.

In some embodiments, the method further includes determining whether ultrasound image data has been acquired for each of the acoustic windows of the ultrasound imaging protocol. If ultrasound image data has been acquired for each of the acoustic windows, the method may further include automatically overlaying onto the external image of the patient, one or more findings graphics, each of which corresponds to an exam finding determined by the ultrasound imaging device based on the acquired ultrasound image data. In some embodiments, each of the one or more findings graphics is selectable, such that responsive to a selection of one of the one or more findings graphics, the method further includes retrieving and displaying at least a portion of the ultrasound image data associated with the selected findings graphic.

In some embodiments, the acquiring and displaying of the external image includes recording and playing back, in real-time, a video of the patient. In some embodiments, the identifying includes applying a deep learning algorithm to one or more frames of the external image to identify the respective locations. In some embodiments, the method involves acquiring a 3D dataset representative of the patient's body shape and generating a 3D model of the patient from the 3D dataset. Some such embodiments of the method may further involve, registering the external image to the 3D model of the patient, and the deep learning algorithm may be trained to use the registered external image and the 3D model for identifying the respective locations.

In some embodiments, the method is implemented, in part, by a computing device which is a hand-held device or a wearable device, and the external image is acquired by a camera integrated into hand-held device or the wearable device. In some embodiments, the ultrasound imaging device is a hand-held imaging device including at least a transducer array, a beamformer, and a signal processor configured to produce the ultrasound image data from echoes detected by the transducer array, wherein the transducer array, the beamformer and the signal processor are all enclosed within a housing of the probe. In some embodiments of the methods described herein, the external image of the patient is displayed in the graphical user interface for providing guidance to the user during the scan without displaying the ultrasound image data acquired during the scan. Embodiments of the present disclosure are further directed to a computer readable medium including instructions which when executed by one or more processors cause the one or more processors to perform a method according to any of the examples herein.

In accordance with further embodiments, disclosed herein are systems and apparatuses for implementing the methods described herein. In some such embodiments, a guidance apparatus may include a camera, at least one processor in communication with the camera; and a memory including instructions which when executed cause the at least one processor to perform a method according to any of the examples herein. In some embodiments, this guidance apparatus provides a graphical user interface according to any of the examples herein. In some embodiments, the guidance apparatus is implemented, at least in part, by a hand-held or a wearable computing device. For example, the camera is built into a tablet or an augmented reality (AR) headset which provide the processor and memory, and wherein the graphical user interface of the guidance apparatus is provided on a display of the tablet or augmented reality headset. In use, the guidance apparatus is communicatively coupled to the ultrasound imaging device. In some embodiments, the ultrasound imaging device is a handheld imaging device, with substantially all components for generating ultrasound images being integrated into the housing of the probe. The handheld imaging device may be communicatively connected (e.g., via a wired or wireless connection) to the hand-held or wearable computing device for communication with the graphical user interface of the guidance apparatus. In some embodiments, the camera of the guidance apparatus may include a stereo camera, a LiDAR optical device, or a combination of the two.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is block diagram of an ultrasound imaging guidance system in accordance with embodiments the present disclosure.
Fig. 2 is a block diagram of a further example ultrasound imaging guidance system in accordance with the present disclosure.
Fig. 3 is a block diagram of another example ultrasound imaging guidance system in accordance with the present disclosure.
Fig. 4 is a block diagram of yet another example of an ultrasound imaging guidance system in accordance with the present disclosure.
Fig. 5 is a flow diagram of a process for guiding a user in an ultrasound imaging procedure in accordance with the present disclosure.
Fig. 6A - 6D are examples of a graphical user interface screen of the guidance interface according to aspects of the present disclosure.
Fig. 6E and 6F are examples of graphical user interface screens of the guidance interface according to aspects of the present disclosure, showing a progress indicator of the guidance interface.
Fig. 6G is yet a further example of a graphical user interface screen of the guidance interface according to aspects of the present disclosure.
Fig. 7 is a block diagram of an example processor according to aspects of the present disclosure.

### DETAILED DESCRIPTION OF EMBODIMENTS

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

A guidance apparatus for guiding a user in an ultrasound imaging procedure may include a camera, at least one processor in communication with the camera, and a memory including instructions executable by the at least one processor. When executed, the instructions cause the guidance apparatus to provide a graphical user interface (GUI) according to any of the examples herein. The graphical user interface (e.g., the one or more graphical user interface screens displayed by the apparatus) may also be referred to herein as guidance interface or graphical user interface. In some embodiments, the guidance apparatus is implemented, at least in part, by a hand-held or a wearable computing device. For example, the guidance apparatus may be provided by a tablet or an augmented reality (AR) headset which provide the camera, processor and memory of the guidance apparatus, and the guidance graphical user interface is provided on a display of the tablet or augmented reality headset. In use, the guidance apparatus is communicatively coupled via a wired or wireless connection to the ultrasound imaging device, which in some embodiments is a handheld imaging device. The term handheld imaging device implies that substantially all components for generating ultrasound images from the echoes detected by the probe are integrated into (or contained within) the housing of the probe. The ultrasound (e.g., handheld) imaging device is communicatively connected (e.g., via a wired or wireless connection) to the hand-held or wearable computing device for communication with the graphical user interface of the guidance apparatus. In some embodiments, the camera of the guidance apparatus may include a stereo camera, a LiDAR optical device, or a combination of the two.

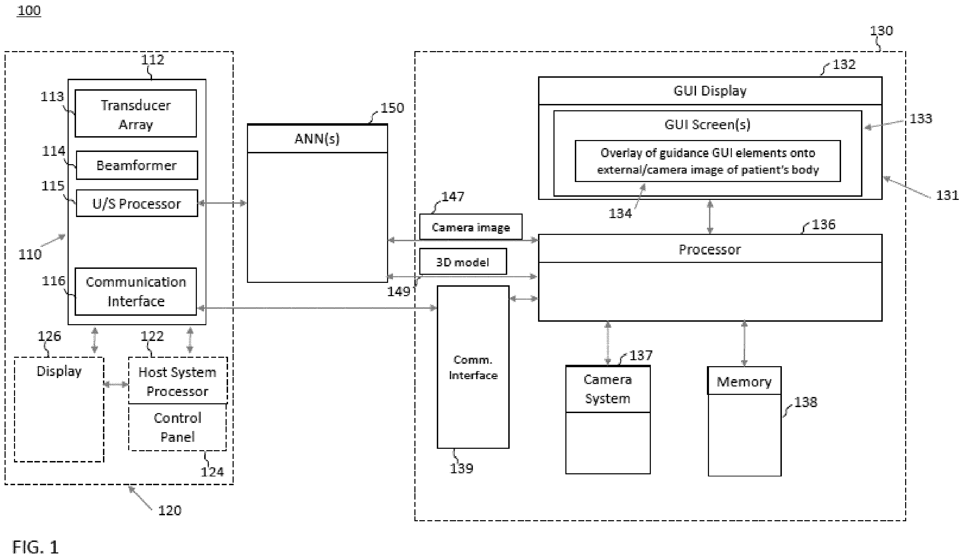

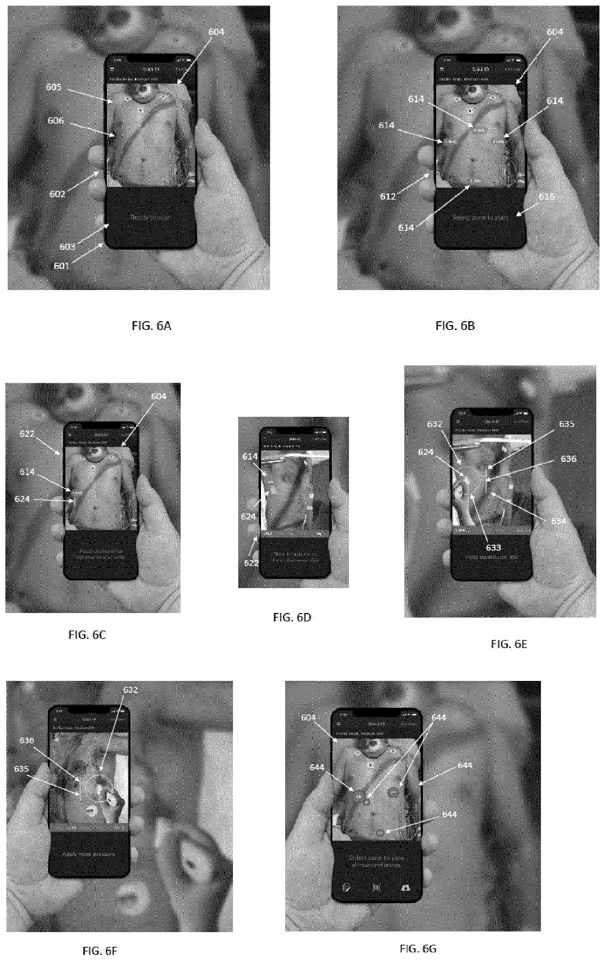

Fig. 1 shows a block diagram of a system 100 including an ultrasound imaging guidance apparatus 130 according to some embodiments of the present disclosure. As shown in Fig. 1, the guidance apparatus 130 includes a camera system (or simply camera) 137 for recording an external image 147 (e.g., a video) of the patient. In some embodiments, the camera 137 and/or additional cameras may be used to acquire an external 3D dataset (e.g., point could data) of the patient's body. The processor 136 may create 3D model 149 (see e.g., mesh 606 in Fig. 6A) of the patient's body based on the 3D dataset 149. This external 3D dataset, also referred to as external 3D scan, can be acquired by a depth camera (e.g., a stereo camera) or another suitable image capture device, such as a LiDAR device. In some embodiments, the 3D dataset may be created from a sequence of external images (e.g., the frames of a video) of the patient. The external images may be registered (e.g., by the processor 136 of the guidance apparatus 130) to the external 3D scan and/or model, such that relative locations in the images can be correlated to those in the 3D scan and/or model, and vice versa.

The guidance apparatus 130 also includes non-volatile memory 138, a processor 136, and a graphical user interface (GUI) 131 including a graphical user interface display 132. Responsive to control by the processor 136, the various graphical user interface screens 134 of the guidance interface 133 (e.g., the one or more overlays of guidance elements onto an external/camera image 147 of the patient) are displayed on the graphical user interface display 132 as will be further described. The memory 138 stores information associated with the guidance interface, such as the images acquired by camera 137, any 3D scans of the patient's body utilized by the system, as well as executable instructions that cause the processor to implement the methods associated with the guidance interface described herein. In use, the guidance apparatus 130 is communicatively coupled to an ultrasound imaging device 110 via a wired or wireless connection. To that end, the guidance apparatus 130 includes a suitable communication interface 139, such as a WiFi communication interface or other suitable wireless communication interface. In some embodiments, the communication interfaces 139 is a standardized wired (e.g., USB, mini DisplayPort, mini HDMI) interface or a proprietary wired communication interface. In embodiments herein, the camera 137, the graphical user interface display 132, the processor 136, the memory 138 and the communications interface 139 are integrated into a single computing device, such as a hand-held computing device (e.g., a smart phone/tablet) or a wearable computing device (e.g., an augmented reality (AR) headset). The guidance apparatus 130 (e.g., processor 136) is in communication with one or more artificial neural networks 150. The artificial neural network(s) 150 receive the camera images of the patient and identify the scanning zones associated with a selected scanning protocol. The artificial neural networks 150 may have any suitable architecture in order to be trained to perform the desired task (e.g., zone identification in an external image of a patient). For example, the artificial neural networks 150 may include one or more convolutional neural networks (CNNs), such as deep convolutional neural networks or other types and/or combination of neural networks. In some embodiments, the artificial neural networks 150 may reside on the apparatus 130, e.g., in the form of executable instructions stored in the memory 138, executed by processor 136. In some embodiments, the artificial neural networks 150 are at least partially on a remote storage device (e.g., on a networked server, which may be on the cloud) communicatively connected to the apparatus 130 and/or ultrasound imaging device 110.

The ultrasound imaging device 110 is configured to transmit ultrasound, detect ultrasound echoes and produce ultrasound images (e.g., gray-scale B-mode mages, color Doppler images, etc.) of or relating to the patient's internal anatomy. The ultrasound imaging device 110 includes a transducer array 113 located in the probe 112 and in communication with at least one beamformer 114. The ultrasound imaging device 110 also includes signal and image processing components, collectively illustrated in Fig. 1 as ultrasound (U/S) processor 115, which can produce ultrasound images from the echoes detected by the transducer array 113. The ultrasound imaging device 110 further includes a corresponding communication interface 116 configured to communicatively couple, via the apparatus's communication interface 139, the ultrasound imaging device 110 to the guidance apparatus 130.

In some embodiments, the ultrasound imaging device 110 is a portable or hand-held unit in that all of the components necessary for transmitting ultrasound, detecting the ultrasound echoes and producing the ultrasound image data may be contained within the housing of the probe 112. One example of such a hand-held imaging device is the LUMIFY ultrasound device/probe by PHILIPS. That is, in some embodiments, a hand-held imaging device such as the LUMIFY probe can be connected to any suitable computing device with a display (e.g., a tablet) that runs an imaging application for displaying the images from the probe. In some embodiments, the computing device to which the hand-held probe is connected may be the same computing device that provides the guidance apparatus. Any other similar suitable imaging device may implement the imaging device 110 of the present disclosure. In some embodiments, the ultrasound imaging device 110 may be connected to or be part of a non-hand-held ultrasound imaging system, such as a cart-based ultrasound imaging system 120 that includes a host system processor 122, a control panel 124 for controlling the operation of the array 113 and other components of the system and one or more additional displays 126, e.g., for displaying ultrasound images. In some embodiments, a fully contained, hand-held imaging device may communicatively couple to an ultrasound host system (e.g., a cart-based ultrasound system), for enhancing the capabilities of the hand-hand imaging device (e.g., with more memory, faster processing, a larger display, etc.).

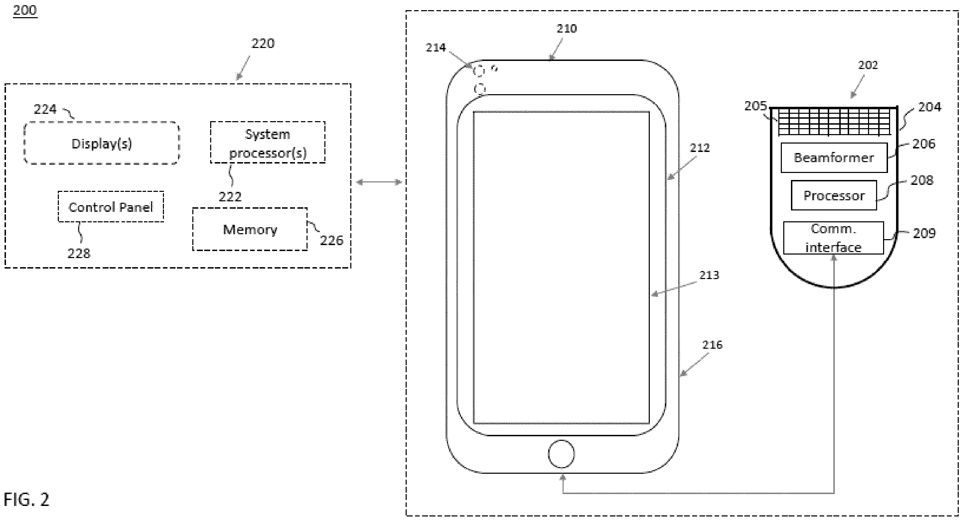

Figs. 2-4 show example implementations of a system including ultrasound imaging guidance according to the present disclosure. Any of the example systems in Figs. 2-4 may be used to implement the system 100. In further embodiments of the present disclosure, one or more of the elements of one of the examples in Figs. 2-4 may be used in combination with elements in another of these examples to implement the system 100. For example, Fig. 2 shows an imaging system with guidance 200, in which the ultrasound imaging device 202 is hand-held unit (e.g., having the electronic components for producing ultrasound image data substantially fully integrated into the housing of the probe 204). The ultrasound imaging device 202 (i.e. probe 204) is configured to provide ultrasound images to any suitable computing device with a display, shown here as the hand-held computing device 210. To that end, the probe 204 includes within its housing the appropriate beamformer 206 and signal processing 208 circuits for generating ultrasound (e.g., B-mode, M-mode, Color Doppler, etc.) images from the echoes detected by the transducer array 205. As shown in Fig. 2, the hand-held computing device 210 may be a tablet computer (or simply tablet) with a display screen 212 of any suitable size. In the context herein, the term tablet refers to any hand-held (or mobile) computing device with a touch screen display (i.e. including smartphones), where the touch screen display is the primary I/O (Input/Output) component of the computing device and substantially defines the size of the computing device. Consequently, the term tablet and smartphone may thus be interchangeably used herein.

The hand-held computing device (e.g., tablet) 210 is further equipped with a camera system 214, a memory and a processor, all enclosed in a single housing 216. The camera system 214 may be a depth camera (also referred to as a stereo camera) including a set (typically two) of cameras operatively arranged to triangulate the 3D location of an object within the field of views of the individual cameras. The display 212 may be a touch screen display which is configured to display a graphical user interface 213 (e.g., the guidance graphical user interface described herein). The hand-held computing device (e.g., tablet) 210 further includes a communications interface configured to communicatively couple with the communications interface 209 of the probe 204. Optionally, the probe 204 and the hand-held computing device (e.g., tablet) 210 may communicate, individually or, in some cases, the probe 204 communicating via the device 210, with a host system 220, which includes additional system processor(s) 222, display(s) 224 and/or memory 226. The host system 220 may be a cart-based ultrasound system, which may also include its own control panel 228 and may be operable for imaging independently of the device 210 and probe 204, for example by coupling any other suitable ultrasound probe thereto.

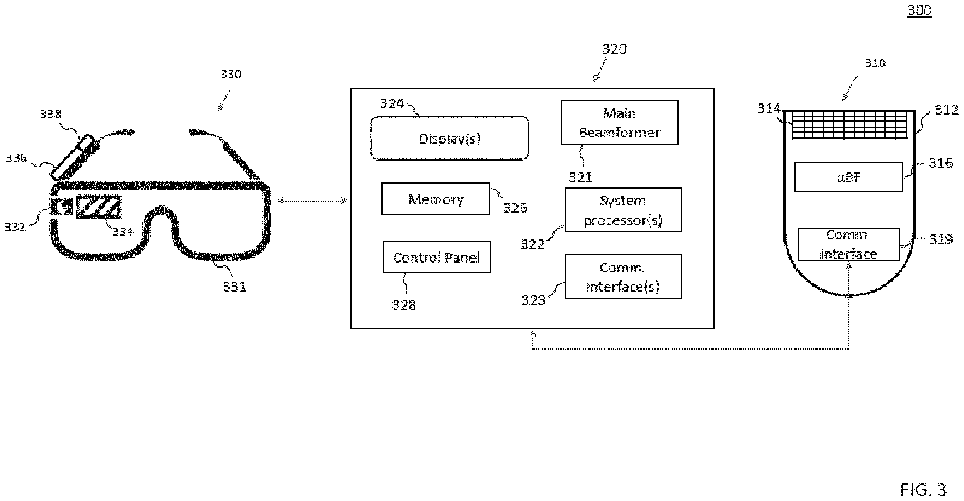

Fig. 3 shows a further example of a system 300 according to the present disclosure. In this example, the ultrasound imaging device 310 includes a probe 312 having a transducer array 314. The probe 312 may be configured as a hand-held imaging device similar to the device 210 of Fig. 2. In such embodiments, the probe may thus contain the beamformer and signal processing circuitry for producing ultrasound image data from the echoes detected by the array 314. In some embodiments, the imaging device 310 is an imaging device that operates in conjunction with beamforming and signal processing circuits in a host system 320. In some such embodiments, the imaging device 310 may optionally include a micro-beamformer 316 which performs partial beamforming (e.g., for reducing the channels of data to be communicated to the host). The partially beamformed signal is communicated, via communication interface 319, to the host system 320 for further processing and generation of the ultrasound image data. The host system 320 may be a cart-based ultrasound scanner which includes a main beamformer 321, one or more host system processors 322, one or more communication interfaces 323 (e.g., for connecting to the imaging device 310 and the guidance apparatus 330), one or more display(s) 324 (e.g., a touch display, a passive non-touch display, or both), memory 326 and a control panel 328 for operating the system 320.

The system 300 further includes a guidance apparatus 330, implemented at least in part by the wearable computing device 331. The wearable computing device 331 includes a camera system (or simply camera) 332, a display 334 for providing the graphical user interface of the guidance system, an on-board processor 336 that drives the display 334 and controls the camera, and a wireless communications interface (e.g., a WiFi or Bluetooth communication interface) 338. In some embodiments, the wearable computing device 331 may be implemented by an augmented (or virtual) reality headset (e.g., the HOLOLENS provided by Microsoft or any other suitable augmented reality / virtual reality headset). As described, the camera may record and stream live video of the patient on the headset display, onto which the graphical user interface elements of the guidance interface are overlaid in real time. In some embodiments, the wearable computing device (e.g., augmented reality / virtual reality headset) 331 is optionally equipped with a microphone for receiving voice commands, for example for user selection of the scanning protocol, for selecting among the multiple scanning zones of the protocol once identified on the displayed image, and/or for the quick retrieval of the ultrasound images associated with any determined medical findings.

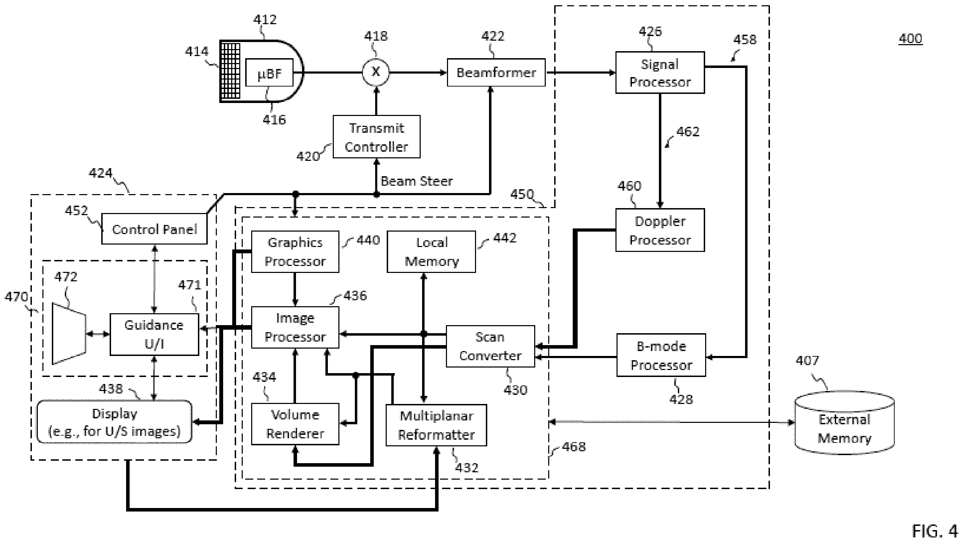

Fig. 4 shows yet another example implementation according to the present disclosure. Shown in Fig. 4 are components of an ultrasound imaging system 400, which may be implemented as a cart-based ultrasound system, and which is operatively associated with a guidance apparatus 470 according to the present disclosure. The system 400 includes a probe 412 including a transducer array 414. The transducer array 414 is configured to transmit ultrasound (e.g., beams, waves) and receive echoes responsive to the ultrasound. A variety of transducer arrays may be used, e.g., linear arrays, curved arrays, or phased arrays. The transducer array 414, for example, can include a two-dimensional array of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some embodiments, the transducer array 414 may be coupled to a micro-beamformer 416, which may be located in the ultrasound probe 412, and which may control the transmission and reception of signals by the transducer elements in the array 414. In some embodiments, the micro-beamformer 416 may control the transmission and reception of signals by active elements in the array 414 (e.g., an active subset of elements of the array that define the active aperture at any given time). In some embodiments, the micro-beamformer 416 may be coupled, e.g., by a probe cable or wirelessly, to a transmit/receive (T/R) switch 418, which switches between transmission and reception and protects a main beamformer 422 from high energy transmit signals. In some embodiments, for example in portable ultrasound systems, the T/R switch 418 and other elements in the system can be included in the ultrasound probe 412 rather than in the ultrasound system base, which may house the image processing electronics. An ultrasound system base typically includes software and hardware components including circuitry for signal processing and image data generation as well as executable instructions for providing a user interface (e.g., processing circuitry 450 and at least a portion of the user interface 424).

The transmission of ultrasonic signals from the transducer array 414 under control of the micro-beamformer 416 is directed by the transmit controller 420, which may be coupled to the T/R switch 418 and the main beamformer 422. The transmit controller 420 may control the direction in which beams are steered. Beams may be steered straight ahead from (orthogonal to) the transducer array 414, or at different angles for a wider field of view. The transmit controller 420 may also be coupled to a user interface 424 and receive input from the user's operation of a user control. The user interface 424 may include one or more input devices such as a control panel 452, which may include one or more mechanical controls (e.g., buttons, encoders, etc.), touch sensitive controls (e.g., a trackpad, a touchscreen, or the like), and/or other known input devices.

In some embodiments, the partially beamformed signals produced by the micro-beamformer 416 may be coupled to a main beamformer 422 where partially beamformed signals from individual patches of transducer elements may be combined into a fully beamformed signal. In some embodiments, micro-beamformer 416 is omitted, and the transducer array 414 is under the control of the main beamformer 422 which performs all beamforming of signals. In embodiments with and without the micro-beamformer 416, the beamformed signals of the main beamformer 422 are coupled to processing circuitry 450, which may include one or more processors (e.g., a signal processor 426, a B-mode processor 428, a Doppler processor 460, and one or more image generation and processing components 468) configured to produce an ultrasound image from the beamformed signals (e.g., beamformed radiofrequency (RF) data).

The signal processor 426 may be configured to process the received beamformed RF data in various ways, such as bandpass filtering, decimation, I (in-phase) and Q (quadrature) component separation, and harmonic signal separation. The signal processor 426 may also perform additional signal enhancement such as speckle reduction, signal compounding, and noise elimination. The processed signals (also referred to as I and Q components or IQ signals) may be coupled to additional downstream signal processing circuits for image generation. The IQ signals may be coupled to a number of signal paths within the system, each of which may be associated with a specific arrangement of signal processing components suitable for generating different types of image data (e.g., B-mode image data, Doppler image data). For example, the system may include a B-mode signal path 458 which couples the signals from the signal processor 426 to a B-mode processor 428 for producing B-mode image data.

The B-mode processor can employ amplitude detection for the imaging of structures in the body. The signals produced by the B-mode processor 428 may be coupled to a scan converter 430 and/or a multiplanar reformatter 432. The scan converter 430 may be configured to arrange the echo signals from the spatial relationship in which they were received to a desired image format. For instance, the scan converter 430 may arrange the echo signal into a two-dimensional (2D) sector-shaped format, or a pyramidal or otherwise shaped three-dimensional (3D) format. The multiplanar reformatter 432 can convert echoes which are received from points in a common plane in a volumetric region of the body into an ultrasonic image (e.g., a B-mode image) of that plane, for example as described in U.S. Pat. No. 6,443,896 (Detmer). The scan converter 430 and multiplanar reformatter 432 may be implemented as one or more processors in some embodiments.

A volume renderer 434 may generate an image (also referred to as a projection, render, or rendering) of the 3D dataset as viewed from a given reference point, e.g., as described in U.S. Pat. No. 6,530,885 (Entrekin et al.). The volume renderer 434 may be implemented as one or more processors in some embodiments. The volume renderer 434 may generate a render, such as a positive render or a negative render, by any known or future known technique such as surface rendering and maximum intensity rendering.

In some embodiments, the system may include a Doppler signal path 462 which couples the output from the signal processor 426 to a Doppler processor 460. The Doppler processor 460 may be configured to estimate the Doppler shift and generate Doppler image data. The Doppler image data may include color data which is then overlaid with B-mode (i.e. grayscale) image data for display. The Doppler processor 460 may be configured to filter out unwanted signals (i.e., noise or clutter associated with non-moving tissue), for example using a wall filter. The Doppler processor 460 may be further configured to estimate velocity and power in accordance with known techniques. For example, the Doppler processor may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some embodiments, the velocity and/or power estimates may undergo further threshold detection to further reduce noise, as well as segmentation and post-processing such as filling and smoothing. The velocity and/or power estimates may then be mapped to a desired range of display colors in accordance with a color map. The color data, also referred to as Doppler image data, may then be coupled to the scan converter 430, where the Doppler image data may be converted to the desired image format and overlaid on the B-mode image of the tissue structure to form a color Doppler or a power Doppler image. In some examples, the scan converter 430 may align the Doppler image and B-mode image.

Outputs from the scan converter 430, the multiplanar reformatter 432, and/or the volume renderer 434 may be coupled to an image processor 436 for further enhancement, buffering and temporary storage before being displayed on an image display 438. A graphics processor 440 may generate graphic overlays for display with the images. These graphic overlays can contain, e.g., standard identifying information such as patient name, date and time of the image, imaging parameters, and the like. For these purposes the graphics processor may be configured to receive input from the user interface 424, such as a typed patient name or other annotations. The user interface 424 can also be coupled to the multiplanar reformatter 432 for selection and control of a display of multiple multiplanar reformatted (MPR) images.

The ultrasound imaging system 400 may include local memory 442. Local memory 442 may be implemented as any suitable non-transitory computer readable medium (e.g., flash drive, disk drive). Local memory 442 may store data generated by the ultrasound imaging system 400 including ultrasound images, executable instructions, training data sets, and/or any other information necessary for the operation of the ultrasound imaging system 400. Although not all connections are shown to avoid obfuscation of Fig. 4, local memory 442 may be accessible by additional components other than the scan converter 430, multiplanar reformatter 432, and image processor 436. For example, the local memory 442 may be accessible to the graphics processor 140, transmit controller 420, signal processor 426, user interface 424, etc. In some embodiments, the system 400 may include or be communicatively connected to external memory 407 (e.g., a storage device of a medical facility's picture archiving and communication system (PACS) or a cloud storage device). Components of the system (e.g., any of the one or more artificial neural networks of the system) may reside in the local memory 442, in the external memory 407, or distributed among the local and external memory of the system.

As mentioned previously ultrasound imaging system 400 includes user interface 424. User interface 424 may include a display 438 and control panel 452. The display 438 may include one or more display devices implemented using any suitable display technology, such as LCD, LED, OLED, or plasma display technology. In some embodiments, display 438 may include multiple displays. The control panel 452 may be configured to receive user inputs (e.g., selection of exam type, imaging mode, imaging settings, etc.). The control panel 452 may include one or more hard controls (e.g., microphone/speaker, buttons, knobs, dials, encoders, mouse, trackball or others). Hard controls may sometimes be referred to as mechanical controls. In some embodiments, the control panel 452 may additionally or alternatively include soft controls (e.g., graphical user interface control elements, or simply graphical user interface controls such as buttons and sliders) provided on a touch sensitive display. In some embodiments, display 438 may include a touch sensitive display (or touch screen) that provides the one or more soft controls of the control panel 452. The imaging system 400 may include a guidance apparatus 470 which includes at least a camera 472 and a guidance user interface (U/I) 471. As described herein, the camera 472 acquires external images of the patient, which are displayed in the guidance U/I 471 overlaid with various graphical user interface elements that provide scanning guidance to the user. The guidance apparatus 470 may operate under the control of a processor contained in the host (e.g., one or more of the processors 468, or the guidance apparatus 470 may have a dedicated processor that controls the camera 472 and/or the guidance U/I 471.

In some embodiments, various components shown in Fig. 4 may be combined. For instance, in some examples, a single processor may implement multiple components of the processing circuitry 450 (e.g., image processor 436, graphics processor 440). In some embodiments, various components shown in Fig. 4 may be implemented as separate components. For example, signal processor 426 may be implemented as separate signal processors for each imaging mode (e.g., B-mode, Doppler, Shear Wave Elastography). In some embodiments, one or more of the various processors shown in Fig. 4 may be implemented by general purpose processors and/or microprocessors configured to perform the specified tasks. In some embodiments, one or more of the various processors may be implemented as application specific circuits. In some embodiments, one or more of the various processors (e.g., image processor 436) may be implemented with one or more graphical processing units (GPU).

As previously noted, an ultrasound imaging guidance apparatus according to the present disclosure is configured to provide guidance, via its graphical user interface (GUI), during an ultrasound imaging procedure, such as to make it easier and/or faster for a physician to perform an imaging protocol (e.g., a FAST imaging protocol or another POCUS imaging protocol). The processor of the guidance apparatus is configured (e.g., via executable instructions) to perform one or more methods associated with providing the guidance interface, examples of which are described further below. An exemplary guidance apparatus may be configured to guide a user in the performance of a particular protocol (e.g., FAST), or optionally it may enable the user to select from among a number of protocols (e.g., via a user control provided in the graphical user interface), responsive to which the apparatus may detect the appropriate set of scanning zones and apply the appropriate machine learning algorithm(s) associated with the selected protocol. In some embodiments, a method executed by a processor of the guidance apparatus for providing guidance for an ultrasound imaging procedure involves acquiring at least one external image of a patient with a camera of a computing device (e.g., the guidance apparatus), which is communicatively coupled to an ultrasound imaging device including a probe. The method further involves identifying, in the external image, respective locations, each associated with one or more acoustic windows for an ultrasound imaging protocol. In some embodiments, the method further involves displaying, on a graphical user interface (GUI) of the computing device, the external image overlaid with a corresponding scanning zone label for each of the respective locations and for each of the respective locations. In some embodiments, upon selection of a given location, the method further involves sequentially displaying: (1) a probe placement graphic corresponding to a position of the probe's face onto the patient's body for acquiring a target view in the corresponding acoustic window, and (2) upon detecting acoustic coupling between the probe and the patient, at least one scanning guidance graphic which is updated in real-time based, in part, on live ultrasound image data acquired by the ultrasound imaging device.

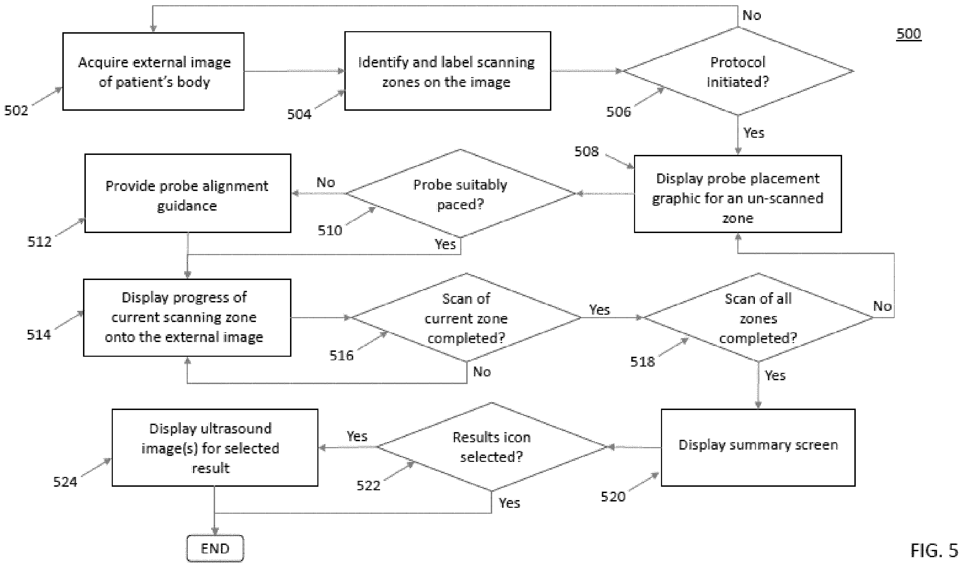

Referring now to Fig. 5, a flow diagram of an example method 500 implemented by a guidance apparatus according to the present disclosure is shown and will be described in further details with reference also to the example guidance graphical user interface displays (e.g., screen captures) shown in Figs. 6A-6G. As noted above, the method 500 may be embodied in executable instructions, which when executed by the one or more processor of a guidance apparatus (e.g., apparatus 130 or 470, computing device 210 or 330) performs the steps of method 500. As shown in Fig. 5, an external image of the patient's body is acquired (see block 502). The external image is acquired by the camera of the guidance apparatus (e.g., the camera of a tablet or an augmented reality headset). As such, the external image captures a picture/video (e.g., typically color/RGB video) of the patient's body from which information about the patient's body habitus (e.g., body type, shape, size, etc.) can be extracted (e.g., via a suitably trained deep learning algorithm). The external image (e.g., one or more frames of the video) is provided to a trained artificial neural network (ANN) to identify the one or more locations in the image (i.e. on the patient's body) associated with the acoustic windows of the selected scanning protocol. The external image may be live video of the subject and the image (e.g., video) may be displayed (e.g., played back in real time in the case of video) on the display of the guidance apparatus (e.g., as shown in Fig. 6A). In the example graphical user interface screen 602, shown in Fig. 6A, the external image 604, which includes the subject, such as a patient's body (or portion thereof) 605 within the camera's field of view, is displayed in real time on the display 603 of the guidance apparatus 601, in the present example a tablet. The external image (or interchangeably "camera image") 604 serves as the guidance interface during the imaging procedure. In other words, in some embodiments only the external image 604 of the subject, such as a patient, is displayed and the guidance markers or graphics are provided as overlays onto the external image (see Figs. 6B-6F), without displaying the ultrasound images acquired during the imaging procedure. This can be advantageous in emergency scenarios, where speed is of the essence and/or the user scanning the subject, such as a patient, is less experienced at locating the proper acoustic windows via the ultrasound images. Another advantage of not having to display the underlying ultrasound image data may be the limited display size (e.g., in the case of hand-held or wearable devices serving as the guidance apparatus). In such cases, displaying only the external images with guidance and not having to also display the ultrasound images may provide for a less cluttered, more user friendly interface, where the image can be maximized on the display and the need to toggle between different images may be obviated.

In some embodiments, the method may optionally involve generating a 3D model of the subject's body. The 3D model may be created from a 3D dataset (e.g., point cloud data) representative of the subject's body shape. The 3D dataset may be acquired by a stereo camera or other suitable imaging device (e.g., LiDAR). In some embodiments, a 3D scanner such as a LiDAR device or other may scan the subject's body to record the 3D dataset. In other embodiments, the point cloud data is extracted from a plurality of image frames taken from different look direction (e.g., the frames of the video being recorded of the subject by the guidance apparatus as the physician visually examines the subject's body). The 3D model (e.g., mesh 606) and the underlying external images (e.g., image 604) may be co-registered and optionally displayed together in the guidance graphical user interface (e.g., as shown in Fig. 6A). In some embodiments, the 3D model (e.g., mesh 606) is not displayed but may be used in the background (e.g., by the guidance algorithm) to identify and track on the display the identified scanning zones and probe placement markers. As described herein, the artificial neural network (e.g., a deep learning algorithm or network) may be trained to use both the external image (e.g., one or more frames of a video) and the 3D model to the identify the locations in the image that correspond to the scanning zones.

As shown in block 504 in Fig. 5, the scanning zones for a given imaging protocol are identified, using and on the external image, and labeled onto the image (e.g., see example scanning zone graphics or labels 614 in the example graphical user interface screen 612 in Fig. 6B). In other words, the external image itself, without any ultrasound image data, is used to identify the one or more primary ultrasound acoustic windows (or scanning zones) associated with a given ultrasound imaging protocol (e.g., FAST examination). For example, in the case of FAST, the primary ultrasound acoustic windows (or scanning zones) include a right upper quadrant (RUQ) view or zone, a left upper quadrant (LUQ) view or zone, a pericardial view or zone (also referred to as subcostal (Sub.) view or zone), and a pelvic view or zone (also referred to as suprapubic (Sub.) view or zone). In some cases, for example in the extended FAST (or eFAST) protocol, additional views (e.g., anterior left and right thoracic views to image the pleura) are recommended and thus additional scanning zones are identified by the processor. In other embodiments, for other imaging protocols a different set of acoustic windows may be used and thus a different set of scanning zones are identified with respect to the subject's body represented in the external image. In some embodiments, the user interface is configured to enable (e.g., via a touch control, physical button or voice command) to select a desired scanning protocol from among different available ultrasound imaging protocols, and based on the selected protocol, the guidance system determines the appropriate set of scanning zones, labeling each zone in the displayed optical image. Continuing with the FAST example, and referring to Fig. 6B, the guidance system may identify the four recommended scanning zones RUQ, LUQ, Sub. and Sup., and may label each zone, via respective scanning zone label 614, at their respective locations on the image 604 displayed in the guidance graphical user interface. In other words, each of the identified zones (or acoustic windows) are identified as locations on the external image 604 of the subject's body and are labeled via a respective scanning zone label 614. The identification of the scanning zones may be performed by any suitable algorithm, such as a properly trained machine learning algorithm. For example, labeled training images of different subjects may be fed to an artificial neural network (ANN) of any suitable architecture (e.g., a convolutional neural network (CNN), a deep convolutional neural network or other suitable deep learning algorithm) the train the artificial neural network to identify the different acoustic windows or zones relative to the subject's body and label them on the image. In some embodiments, the graphical user interface may be further configured to provide additional instructions to the user, such as by display a text instruction 616 adjacent to the image 604 and/or by providing these instructions audibly (e.g., via a speaker of the augmented reality headset or tablet).

Returning to Fig. 5, when the scanning protocol is initiated (see decision block 506 in Fig. 5), the guidance interface automatically displays a probe placement graphic or marker 624, corresponding to a selected scanning zone (see block 508). In some embodiments, the selection of a scanning zone may occur automatically, for example by the processor selecting a first zone recommended to be scanned per the scanning protocol upon receipt of an indication to initiate the protocol. Similarly, and upon completion of the scan for each zone, the processor may select the subsequent zones of the protocol automatically by determining which zones have not been scanned yet and then selecting one of the un-scanned zones (e.g., randomly or based on a predetermined preferred order). In some embodiments, the selection of one or more of the zones may be responsive to user input. Thus, in the case of the first selected zone, the protocol may be initiated by the user selecting a specific one of the identified zones. To that end, in some such embodiments, each of the scanning zone labels 614 in the graphical user interface display of Fig. 6B is a selectable graphical element of the graphical user interface configured to enable the user to select a corresponding location/zone to initiate the subsequent sequential displaying of guidance elements.

The probe placement marker 624 is displayed concurrently with the external image 604 in the guidance graphical user interface, and more specifically the probe placement marker 624 is displayed onto (i.e. overlaid on) the external image 604 (as shown, e.g., in the example graphical user interface screen 622 of Fig. 6C). As previously mentioned, the external image 604 may be live video of the patient being recorded and played back in real time, in which case the location of the probe placement marker 624 on the image may dynamically adjust as the look direction, and consequently the portion of the patient's body in the field of view, changes. This can be seen, for example in Fig. 6D, which shows another screen shot of the graphical user interface 622 of Fig. 6C, which includes the probe placement marker 624 overlaid onto the correct location of the patient's body even though the underlying image changed due to a change of viewing (or look) direction of the camera. To avoid cluttering the display, which may be particularly challenging on a small sized display of a hand-held or wearable device, upon selection of one of the number of scanning zones, the scanning zone labels of the non-selected zones may be removed from the graphical user interface, optionally leaving only the scanning zone label 614 for the selected zone (as shown in Figs. 6C and 6D). The probe placement marker 624 may have a shape corresponding generally to the face of the probe such that it provides a visual indication (or guidance) to the user on how to properly place/align, on the specific patient's body, the face of the probe in order to acquire the target view(s) associated with the selected acoustic window. As such, and because the guidance is provided as an overlay onto the external image of the patient's body, the probe placement markers may enable the user to more easily and faster position the probe at the appropriate location on the patient's body to acquire the target view(s). As previously noted, the external image may be live video, in which case the guidance interface dynamically adjusts the graphical user interface display (e.g., adjusting the position/shape of the probe placement marker) to track its identified location as the user changes the look direction and thus the field of view of the camera. As also mentioned, this tracking may be enabled, in part, by a 3D model of the patient's body that may be co-registered with the RGB images.

In some embodiments, the guidance system may determine if the probe is properly placed/aligned to acquire the target view (see decision block 510) and may provide guidance for aligning the probe (see block 512). In some embodiments, after the probe is contacted to the patient, the guidance system may use the live ultrasound image data acquired by the probe in assessing, in real time, the alignment of the probe and providing guidance. In some embodiments, the guidance system may additionally or alternatively use the external image (e.g., live video) to provide the probe alignment guidance. In the case of the former, for example, the real-time acoustic data detected by the probe may be coupled to a deep learning algorithm which can detect, firstly, whether the probe is coupled to the patient and can, then, detect, whether the field of view of the probe is aligned with one or more desired view(s). If the live image detected by the probe at its current position does not correspond to the target view, the graphical user interface may provide probe placement guidance to aid the user in suitably placing or aligning the probe. This ultrasound data evaluation may occur entirely in the background without displaying any of the ultrasound images onto the guidance display. As noted, the system may additionally or alternatively use the external image to make or enhance the determination of proper alignment. In some embodiments, the system may additionally and optionally determine, using the ultrasound image data, whether sufficient pressure is being applied with the probe and the system may graphically provide guidance (e.g., via a pressure graphic 635 in the example graphical user interface screen in Fig. 6F) for example, to instruct the user to increase pressure. Probe alignment guidance based on the acquired ultrasound images and/or external, camera images may be generated and provided in accordance with any of the examples in commonly-owned U.S. Patent Application No. 62/746,042 titled "Deep Learning-Based Ultrasound Imaging Guidance And Associated Devices, Systems, and Methods," filed October 16, 2018 (corresponding to WO 2020/079077), and U.S. Patent Application No. 62/985,596, titled "Ultrasound Imaging Guidance And Associated Devices, Systems, and Methods," filed March 5, 2020 (corresponding to WO 2021/175965).

In some embodiments, the probe placement marker 624 may provide guidance through its animation. For example, the probe placement marker 624 may pulsate (or flicker) to indicate that proper alignment of the probe's field of view with the target view for selected acoustic window has not been achieved. In other words, as the user is manipulating the probe on the patient's body to properly align the probe, the marker 624 may pulsate until the system detects that alignment of the probe's field of view with the target view has been achieved. In some embodiments, rather than pulsating, the probe placement marker 624 may change color upon detecting proper alignment, for example from blue or another color to green (e.g., as shown in Fig. 6E) or any other pre-determined color. In some embodiments, the probe placement marker 624 may both change color and stop pulsating upon detection of proper alignment. In yet other embodiments, the pulsating animation may be used to indicate further need for alignment, while the changing of color may indicate that the system has detected acoustic coupling between the probe and the patient's body. Any suitable algorithm may be used to determine whether the probe is properly aligned, such as a deep-learning network trained on training sets of ultrasound image data and/or external (camera) images of different patients and different imaging probes. The alignment guidance may be provided by any suitable means, such as graphically, audibly and/or via tactile feedback. Graphically, the alignment guidance may be provided in accordance with any of the examples the aforementioned US patent applications 62/746,042 and 62/985,596. In some embodiments, the alignment guidance may include instructions (e.g., graphics) which instruct the user how to move the probe (e.g., tilt, slide, rotate) in order to suitably position the probe for acquiring the target view. The determination(s) that drive the probe alignment guidance and/or the animation of the probe placement marker 624 may be made, in some embodiments, using any suitable and properly trained artificial neural network. In some embodiments of method 500, steps 510 and 512 may be omitted.

The method may involve determining whether sufficient image data has been acquired for the current scanning zone. The system may do this automatically, in the background, in some cases using a machine learning algorithm, which compares the acquired image data to an expected image volume (e.g., a single or multiple views) for the current zone. While the system performs the sufficiency analysis, the graphical user interface may concurrently display a graphical indicator of the sufficiency of the scan. For example, and referring back to Fig. 5, after the probe is placed in contact with the patient (i.e., acoustically coupled to one of the acoustic windows), the method 500 may proceed with the imaging system acquiring ultrasound image data while the guidance apparatus processes and determines, in the background, whether sufficient image data for the current scanning zone has been acquired (decision block 516) and displays the progress (e.g., via a progress graphic or indicator 632) onto the graphical user interface display (see block 514). The indication of progress is provided via the guidance graphical user interface (e.g., onto the external image 604) concurrently, in real time, with the ultrasound scanning. For example, acquired ultrasound image data (e.g., the real-time cine-loop buffer/memory) may be coupled to an artificial neural network, for example instead of, or in some cases in addition to, being coupled to a graphics processor for display. In some embodiments, the image data is not coupled to a display and instead, the image data is used in the background during the scanning process to guide the user, via the external image alone.

The artificial neural network makes the determination of the sufficiency of the acquired image data in real time and outputs a measure of progress (e.g., percent complete) for use by the guidance interface in updating the progress graphic. For example, the artificial neural network may be trained on an expected volume of image data (e.g., a set of one or more views) from a given acoustic window, and the artificial neural network may thus analyze the recorded image data (e.g., the data recorded to the cine-loop buffer/memory) against the expected volume in deciding of the sufficiency of the data and progress towards fully scanning from the given acoustic window. The one or more guidance graphics overlaid onto the external image may thus include a progress indicator graphic, which may be animated to indicate a status of the recording of the expected volume. In some embodiments, the progress indicator 632 may be in the form of a circular progress bar 633 which is arranged on the display so that it encircles the probe placement marker 624 overlaid on the external image. The progress indicator 632 is dynamically updated, in real time, in response to output from the artificial neural network (e.g., based on a percent completed for the current scanning zone). The steps of determining whether the zone is fully scanned (block 516) and the providing of dynamic visual feedback by displaying the progress of the scan (e.g., block 514) are repeated until the scan of the current zone completes (e.g., sufficient image data for making medical findings for that zone has been acquired). In some embodiments (e.g., if so configured and/or selected) the guidance apparatus may automatically make medical findings based on the acquired image data, and the progress indicator (e.g., 632 in Figs. 6E and 6F) may additionally indicate the status or progress of the medical finding's analysis. As such, the progress indicator may function as a timer instructing the user to continue to hold the probe at the current location until the system completes scanning the area and/or analyzing the acquired image data for medical findings.

When all scanning zones have been scanned, as determined at block 518, the system may optionally display a results (or summary) screen (see block 520), an example of which is shown in the graphical user interface screen in Fig. 6G. In some embodiments, the summary screen is displayed automatically upon completion of the protocol (e.g., when the system determines at block 520 that all zones have been scanned). In some embodiments, the summary screen is displayed responsive to user input, thus the graphical user interface, upon completion of the protocol, displays a query to the user on whether the summary screen should be displayed. In this summary screen, diagnostically relevant medical findings, identified based on the acquired image data, are graphically represented (e.g., via one or more medical findings (or results) graphics 644) overlaid onto the external image 604, for example, as shown in Fig. 6G. The medical findings may be identified from the image data using another properly trained artificial neural network. The artificial neural network may be trained to identify various diagnostically relevant findings. For example, in the context of a FAST protocol, the artificial neural network may be trained to identify free fluid (FF), clotted blood (CB) or other diagnostically relevant findings based on the acquired image data. The size of each individual medical finding's graphic may convey information about the severity of the issue. For example, the size of a given graphic 644 may indicate the size of a clot or the volume of free fluid identified from the image data. The medical findings graphics 644 may optionally be selectable (e.g., via a touch selection or via voice command by speaking the label of any given results graphic), responsive to which (see block 522 of Fig. 5) the underlying medical images based on which the determination was made may be automatically retrieved and/or displayed (see block 524 of Fig. 5), e.g., for verification or further review by the clinician. In some embodiments, the blocks 520, 522 and 524 of Fig. 5 may be omitted and the method 500 may, thus, terminate upon determining that all scanning zones have been scanned at block 518. As can be seen, during the entire scan protocol, the user may be guided using solely the external (or camera) image of the patient's body without the user having to interpret medical image data, which can be time consuming and can slow down an emergency imaging procedure.

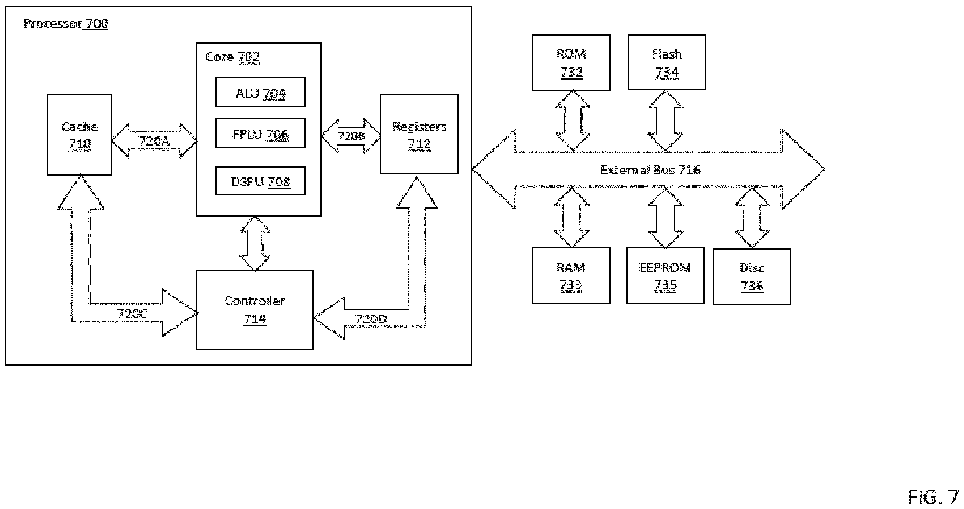

The systems and methods described herein may be implemented by any suitable combination of hardware and software components. In some embodiments, one or more of the methods described herein may be embodied (e.g., as executable instructions) in computer readable medium, which when executed cause one or more processors to perform the methods described. Fig. 7 shows, in block diagram form, components of an example processor 700, which may be used implement one or more of the processors described herein (e.g., processor 116, 136, 208, 222, 332, 436, 440, etc.) Processor 700 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 700 may include one or more cores 702. The core 702 may include one or more arithmetic logic units (ALU) 704, floating point logic units (FPLU) 706, digital signal processing units (DSPU) 708, or any combination thereof. The processor 700 may include one or more registers 712 communicatively coupled to the core 702. The registers 712 may be implemented using dedicated logic gate circuits (e.g., flip-flops) and/or any memory technology. In some embodiments the registers 712 may be implemented using static memory. The register may provide data, instructions and addresses to the core 702. In some embodiments, processor 700 may include one or more levels of cache memory 710 communicatively coupled to the core 702. The cache memory 710 may provide computer-readable instructions to the core 702 for execution. The cache memory 710 may provide data for processing by the core 702. In some embodiments, the computer-readable instructions may have been provided to the cache memory 710 by a local memory, for example, local memory attached to the external bus 716. The cache memory 710 may be implemented with any suitable cache memory type, for example, metal-oxide semiconductor (MOS) memory such as static random access memory (SRAM), dynamic random access memory (DRAM), and/or any other suitable memory technology.

The processor 700 may include a controller 714, which may control input to the processor 700 from other processors and/or components included in a system (e.g., control panel, touch screen interface and/or other processors of the system) and/or outputs from the processor 700 to other processors and/or components included in the system (e.g., a display and/or other processors of system). Controller 714 may control the data paths in the ALU 704, FPLU 706 and/or DSPU 708. Controller 714 may be implemented as one or more state machines, data paths and/or dedicated control logic. The gates of controller 714 may be implemented as standalone gates, FPGA, ASIC or any other suitable technology. The registers 712 and the cache memory 710 may communicate with controller 714 and core 702 via internal connections 720A, 720B, 720C and 720D. Internal connections may be implemented as a bus, multiplexer, crossbar switch, and/or any other suitable connection technology.

Inputs and outputs for the processor 700 may be provided via a bus 716, which may include one or more conductive lines. The bus 716 may be communicatively coupled to one or more components of processor 700, for example the controller 714, cache memory 710, and/or register 712. The bus 716 may be coupled to one or more components of the system, e.g., the graphical user interface of the system. The bus 716 may be coupled to one or more external memories. The external memories may include Read Only Memory (ROM) 732. ROM 732 may be a masked ROM, Electronically Programmable Read Only Memory (EPROM) or any other suitable technology. The external memory may include Random Access Memory (RAM) 733. RAM 733 may be a static RAM, battery backed up static RAM, Dynamic RAM (DRAM) or any other suitable technology. The external memory may include Electrically Erasable Programmable Read Only Memory (EEPROM) 735. The external memory may include Flash memory 734. The external memory may include a magnetic storage device such as disc 736. In some embodiments, the external memories are included in the non-volatile memory of an apparatus, computing device or a system, such as the guidance apparatus 130, ultrasound imaging device 110 or ultrasound imaging system 120 shown in Fig. 1.

A person of skill in the art will understand that the particular embodiments illustrated above are exemplary and are not intended to limit the scope of the present disclosure. In that regard, a variety of modifications, substitutions, and/or combinations could be made with respect to the embodiments described above without departing from the scope of the present disclosure. It will also be understood that one or more of the steps of the method 500 described above may be performed by one or more components of an ultrasound imaging system, such as a processor or processor circuit, a multiplexer, a beamformer, a signal processing unit, an image processing unit, or any other suitable component of the system. For example, one or more steps described above may be carried out by the processor circuit 136 described with respect to Fig. 2. The processing components of the system can be integrated within an ultrasound imaging device, contained within an external console, or may be distributed between various components of the system. Although one or more of the examples of graphical user interfaces, indicators, and representations described above are shown as two-dimensional and may be formatted for two-dimensional display devices, the graphical user interfaces, indicators, and representations described above may also include three-dimensional visualizations formatted for three-dimensional display devices, such as augmented reality devices, virtual reality devices, 3D-capable monitors, etc.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements.

## Claims

1. A method (500) of providing guidance to a user in an ultrasound imaging procedure, the method comprising:
acquiring (502) an external image (604) of a subject with a camera (137, 332, 472) of a computing device (130, 210, 331, 470) communicatively coupled to an ultrasound imaging device (120, 202, 310) comprising a probe (112, 204, 312, 412);
identifying (504), in the external image (604), respective locations, each associated with one or more acoustic windows for an ultrasound imaging protocol;
displaying (508), on a graphical user interface (131, 213) of the computing device, the external image (604) overlaid with a corresponding scanning zone label (614) for each of the respective locations; and
for each of the respective locations, and upon selection of a given location, displaying onto the external image a probe placement graphic (624) corresponding to a position of the probe's face onto the subject's body for acquiring a target view in the corresponding acoustic window.

2. The method (500) of claim 1, wherein each of the scanning zone labels (614) is a selectable graphical element of the graphical user interface (131, 213) configured to enable the user to select the corresponding location to initiate said sequential displaying.

3. The method (500) of claim 1 or 2, further comprising:
animating (510) the probe placement graphic (624) to indicate at least one of: acoustic coupling between the probe and the subject, and alignment of the probe's field of view with the target view,
wherein optionally said animating the probe placement graphic (624) comprises at least one of:
changing a color of the probe placement graphic upon detecting acoustic coupling between the probe and the subject or upon detecting alignment of the probe's field of view with the target view; and
pulsating the probe placement graphic until alignment of the probe's field of view with the target view has been detected.

4. The method (500) of any of the preceding claims, further comprising: upon detecting acoustic coupling between the probe and the subject, displaying (512) at least one scanning guidance graphic onto the external image (604) and updating the at least one scanning guidance graphic in real-time based, in part, on live ultrasound image data acquired by the ultrasound imaging device.

5. The method (500) of claim 4, further comprising: determining (516) whether an expected volume of ultrasound image data from a current acoustic window has been recorded, and wherein the at least one scanning guidance graphic comprises a progress indicator graphic (632) animated to indicate a status of the recording of the expected volume,
wherein optionally the progress indicator graphic (632) comprises a circular progress bar (633) encircling the probe placement marker in the external image, and/or
wherein optionally the at least one scanning guidance graphic further comprises a pressure indicator graphic (635) displayed onto the external image (604) adjacent to the progress indicator graphic (632).

6. The method (500) of any of the preceding claims, further comprising:
determining (518) whether ultrasound image data has been acquired for each of the acoustic windows of the ultrasound imaging protocol; and
if ultrasound image data has been acquired for each of the acoustic windows, automatically overlaying (520) onto the external image (604) of the subject, one or more findings graphics, each of which corresponds to an exam finding determined by the ultrasound imaging device based on the acquired ultrasound image data.

7. The method (500) of claim 6, wherein each of the one or more findings graphics is selectable, and wherein responsive to a selection (522) of one of the one or more findings graphics, the method further comprises retrieving and displaying (524) at least a portion of the ultrasound image data associated with the selected findings graphic.

8. The method (500) of any of the preceding claims, wherein the computing device is a hand-held device (210) or a wearable device (331), and the external image (604) is acquired by a camera (214, 332) integrated into the hand-held device or the wearable device, and wherein optionally said acquiring and displaying of the external image comprises recording and playing back, in real-time, a video of the subject.

9. The method (500) of any of the preceding claims, wherein said identifying comprises applying a deep learning algorithm to one or more frames of the external image (604) to identify the respective locations.

10. The method (500) of any of the preceding claims , further comprises acquiring a 3D dataset representative of the subject's body (605) shape and generating a 3D model (606) of the subject from the 3D dataset, and registering the external image (604) to the 3D model (606) of the subject, wherein the deep learning algorithm is trained to use the registered external image (604) and the 3D model (606) for identifying the respective locations.

11. The method (500) of any of the preceding claims, wherein the ultrasound imaging device (110, 202, 310) is a hand-held imaging device (110, 202, 310) comprising at least a transducer array (113, 205, 314), a beamformer (114, 206), and a signal processor (115, 208) configured to produce the ultrasound image data from echoes detected by the transducer array (113, 205, 314), wherein the transducer array (113, 205, 314), the beamformer (114, 206) and the signal processor (115, 208) are all enclosed within a housing of the probe (112, 204, 312).

12. A computer readable medium comprising instructions which, when executed by one or more processors (700), causes the one or more processors to perform a method according to any of the preceding claims.

13. An ultrasound imaging system (100, 200, 300, 400) comprising:
an ultrasound imaging device (110, 202, 310) comprising a probe (112, 204, 312, 412); and
a guidance apparatus (130, 330, 470) configured to communicatively couple to the ultrasound imaging device, the guidance apparatus comprising:
a camera (137, 214, 332, 472);
at least one processor (136, 336) in communication with the camera; and
a memory (138) comprising instructions which when executed by the at least one processor cause the at least one processor to perform a method according to any of claims 1-11.

14. The ultrasound imaging system (100, 200, 300, 400) of claim 13, wherein at least one of:
the camera (137, 214, 332, 472) comprises a stereo camera, a LiDAR optical device, or a combination of the two; and
the ultrasound imaging device (110, 202, 310) is a handheld imaging device (110, 202, 310) integrated into a housing of the probe (112, 204, 312).

15. The ultrasound imaging system (100, 200, 300, 400) of claim 13 or 14, wherein the camera (137, 214, 332, 472) is built into a tablet or an augmented reality headset, and wherein the graphical user interface is provided on a display of the tablet or augmented reality headset.
